# EUROPEAN PATENT SPECIFICATION

(11) **EP 0 527 961 B1**
(45) Date of publication and mention of the grant of the patent: **17.12.1997**
(21) Application number: 91920938.7
(22) Date of filing: 03.05.1991
(51) Int. Cl.: A61M 25/02, A61M 39/00

(54) **CATHETERIZATION SYSTEM WITH UNIVERSAL RETENTION DEVICE**
KATHETERISIERUNGSSYSTEM MIT UNIVERSALER HALTEVORRICHTUNG
SYSTEME DE CATHETERISME AVEC DISPOSITIF DE FIXATION UNIVERSEL

(30) Priority: 04.05.1990 US 518964
(43) Date of publication of application: 24.02.1993
(73) Proprietor: Venetec International, Inc., Mission Viejo, California 92691 (US)
(72) Inventor: BIERMAN, Steven, F., Del Mar, CA 92014 (US); HOWSON, David, C., Denver, CO 80206 (US)
(74) Representative: W.P. Thompson & Co.
(86) International application number: PCT/US91/03074
(87) International publication number: WO 91/16939

(56) References cited:
- EP-A- 0 114 677
- EP-A- 0 169 704
- GB-A- 2 063 679

## Description

### Background of the Invention

The present invention relates to a percutaneous catheterization system which is universally compatible with standard connectors, and, more particularly, to a system which facilitates the frequent replacement of the catheter tubing which is necessary in such catheterization procedures.

It is very common in the treatment of hospitalized patients to utilize intravenous (IV) catheters to introduce certain fluids directly into the bloodstream of the patient. Such procedures are also becoming more common outside of the hospital as the high cost of hospital medical care has brought about the advent of neighborhood out-patient clinics and home health care.

In IV catheterization, a supply of fluid is maintained in a container which is located at a height higher than the patient. The catheter tubing flows from the supply container to the location of introduction into the patient where it is attached to a catheter. This location is typically the back of the patient's hand or a vessel on the inside of the arm. Typically, a needle or other stylet is first introduced through the cannula portion of the catheter and into the skin of the patient at the desired location, and then removed after the cannula is inserted into the skin. The fluid then flows directly into the blood vessel of the patient by gravity, or, if necessary, by the pressure generated by the head of the fluid above the height of the patient.

In common practice, the catheter is maintained in place on the skin of the patient by the use of adhesive or surgical tape. Likewise, the connection between the tubing and the catheter is also maintained by use of tape. In addition, a safety loop is typically formed in the tubing so that any tension applied to the tubing is not passed directly to the cannula of the catheter but is taken up in the slack of the safety loop. This loop is also typically taped loosely to the skin of the patient. This entire taping procedure takes several minutes of the valuable time of health practitioners performing this procedure.

IV catheterization is frequently maintained for several days, depending upon the condition of the patient. This longevity requirement gives rise to several problems associated with IV catheters. For example, the catheter tubing is generally replaced every 24 to 48 hours in order to maintain the sterility of the fluid and the free-flow of the fluid through the tubing. Thus, a health practitioner is often called upon to frequently change the tubing and to retape the connection. Furthermore, the taping of the catheter to the skin of the patient often covers the location of insertion of the cannula. Thus, the tape must be removed in order to inspect the insertion location for inflammation or infection and a complete taping procedure again be initiated. In short, a great deal of valuable time of the health practitioner is used in applying significant amounts of surgical tape to IV catheters. Further, the frequent application and removal of surgical tape often results in the excoriation of the skin of the patient in the area of the insertion.

A number of catheterization systems have recently been developed which improve the stabilization of the catheter system and/or obviate the need for frequent application and removal of surgical tape during IV tubing changes. One such system is shown in U.S. Patent No. 4,250,880, wherein a disposable catheter stabilizing fitting comprising a catheter hub-retaining cradle is attached to a laminar base, the base having an adhesive undersurface for attachment to the patient. The cradle is designed to hold the catheter hub at a suitable angle to avoid bending or crimping of the catheter so as to facilitate access to the point of catheter insertion to the skin, however, the device is still required to be secured and stabilized by being overlaid with adhesive tape.

Another catheterization system is described in U.S. Patent No. 4,711,636 on which the preambles of claims 1 and 18 are based, comprising a specially designed cannula which is attachable via an adaptor to an IV tubing. The cannula is removably attached to a base, which is adhesively attachable to the skin of a patient. The cannula is adapted to be secured to the base portion in a snap-fit engagement so as to obviate the necessity for the time consuming application of surgical tape or other apparatus to stabilize the catheter, as previously mentioned. However, this cannula is specially adapted for attachment to the base portion of the assembly, and therefore, cannot be used with conventional luer-type connectors on conventional cannula apparatus.

The above-referenced pending parent patent application discloses a system which represents an improvement over the apparatus shown in above-mentioned patent 4,711,636 in that the system in the pending application can be used with conventional luer-type connectors on conventional cannula apparatus. However, one of the difficulties of utilizing an adaptor which can attach to conventional connectors is that there are some dimensional variances in conventional connectors. Typically, such connectors employ a tapered hub with an outwardly extending flange on the larger end. That pending application employs a retention latch which is manually movable to latch onto the flange on the end of the connector, and thus securely hold the connector with respect to the adaptor. This works quite well with most standard-type connectors, but connectors having slight dimensional variations do not fit so well because there is no means in the latch to accommodate such variations. Thus, a need exists for a latch that can handle such variations.

The manually operated mechanism for opening the latch in the above-referenced application and in the above-mentioned patent 4,711,636 extends outwardly away from the patient skin to facilitate operation. While the device is relatively small, it does extend outwardly enough such that it can interfere in taping or other medical procedures, or can interfere with patient movement. Thus it is desirable that a system be provided which improves that aspect.

Another type of standard IV connection involves the use of "injection sites." Although such sites may occur at various locations in IV catheterization systems, one common location is where the catheter is directly inserted into the patient's vein. This injection site connection comprises a cylindrical tube mounted on the end of the catheter with a rubber septum or "buff cap" stretched over or inserted within the open end of the tube. Originally, this type of connection was intended to facilitate manual injections. That is, the nurse can insert a needle through the rubber material of the septum and into the lumen on the other side thereof in order to cause medication to enter the patient. Once the needle is withdrawn, the rubber material of the septum closes the puncture area and seals the lumen. In addition to manual injections, however, this type of septum connector can be left in place in order to permit secondary attachments for IV purposes. The top surface of the septum can be easily cleaned and sterilized with an alcohol-saturated pad. Thus, this type of septum or buff cap connection is an alternative to a catheterization system utilizing a luer lock or hub on the end of the catheter. In the IV context, the septum connection is made up only by the insertion of a needle attached to the end of the IV line which is inserted through the rubber material of the septum.

However, along with the convenience provided by this septum-type connection, a number of problems have also arisen. First, the mechanical security of the connection of a needle inserted through rubber is obviously very poor. This problem is particularly aggravated by the duration of the connection. With the advent of long-term infusion, which has replaced manual injections for many applications, the needle/septum connection remains in use for many hours or days. Furthermore, this type of connection is frequently used in the home healthcare context. Because the patient is often ambulatory, the connection frequently disengages. As a consequence, nurses typically try to tape the connection together. However, this procedure takes extra time and does not protect the connection site from contamination. Also, with more frequent use of IV therapy, nurses are exposed many times each day to sharp needles. This type of septum connection increases the risk of accidental self-injection. Because many of the drugs contained in the IV solution are potent and toxic, this may be a very significant risk to a nurse using a septum connection.

Previous devices have not adequately addressed these problems. Most manufacturers have provided two customized adapters in order to avoid the above problems presented by the septum connection. For example, one component would comprise a special device incorporating the rubber cap and a second customized device incorporates the IV needle. Thus, standard septum connectors cannot be utilized. Furthermore, connector components are not interchangeable with other manufactured components. This two-piece customized approach also increases the expense associated with this form of IV therapy. Furthermore, previous devices do not provide adequate protection against accidental self-injection. The customized components are often bulky and prevent injections close to the patient's skin. Furthermore, these devices do not provide prevention against contamination of the septum surface after the connection is made. In other words, nurses still find it necessary to tape the connection, increasing the expense and time consumption of this procedure.

According to the present invention there is provided an apparatus for connecting a catheter to supply tubing, comprising:
an adaptor having a tubular body with a passage therethrough, the body having a forward end to connect to a catheter end and a rear end adapted to be connected to said tubing;
a latch slidably mounted on said adaptor for movement in a direction generally parallel to an axis through said passage, said latch including a forward portion adapted to cooperate with said catheter end to hold the catheter end on said forward end, said latch and said adaptor having interengaging structure to hold said latch in a manually selected position on said adaptor; and
a base adapted to be mounted on a patient and to support said adaptor, said base and said adaptor having structure to releasably secure said adaptor to said base.

Briefly stated, the invention employs an adaptor which is removably secured to a base adapted to be mounted on a patient. The adaptor has a tubular portion with a forward end compatible with either a standard luer-type connector or a septum-type connector; and a clip slidably mounted on the adaptor to latch the connector to the adaptor. The clip includes a forward latch portion which cooperates with a flange on the rear of the connector to secure the connector to the adaptor. Because the clip is slidable, it can accommodate variations in the axial position of the connector flange or septum configuration. Preferably, the latch has a somewhat flexible forked tip that straddles the end of the connector, and accommodates variations in connector diameters.

In one embodiment, the adaptor body is provided with a forward end adapted to be inserted within the end of a standard luer-type connector. In another embodiment, the invention utilizes an IV needle mounted within and protruding from the forward end of the tubular portion of the adaptor so as to be compatible with a standard septum-type connector. This embodiment also utilizes a shield which substantially prevents the risk of accidental self-injection and reduces the risk of contamination of the septum during connection. In both embodiments, the adapter is streamlined and lightweight in order to avoid discomfort and bulkiness, and to permit intricate injections close to the patient's body. Furthermore, the adapter can be rotated to a low-profile position, in order to enhance the security of the connection. Thus, as an important advantage of this invention, the adapter is compatible with a wide range of standard IV connectors, and the need for special or customized components is eliminated.

In a preferred form of the invention, the clip is mounted on the adaptor by means of an elongated plate-like ratchet element which extends parallel to the adaptor tubular portion. The element is mounted on the end of an arm that extends radially outwardly from the adaptor tubular portion. The clip is slidably mounted on this plate-like ratchet element. Depressing the rear of this element, with the clip-mounted thereon, toward the adaptor tubular portion allows it to pivot about the arm so as to move the forward end of the ratchet element, together with the forked end of the clip, radially outwardly to permit engagement and disengagement of the connector to the end of the adaptor. Withdrawing the depressing force allows the forked end of the clip to return inwardly to its normal latching position.

The clip and the ratchet element include ratchet teeth and an interengaging pawl for maintaining those parts in a manually selected position. In a preferred form of the invention, the ratchet teeth are formed on the radially outer surface of the ratchet element and a tongue-like, resiliently mounted pawl formed on the clip cooperates with these teeth.

According to a further aspect of the present invention, there is provided an apparatus for supporting and facilitating the changing of tubing connected to a catheter, comprising an adaptor having a tubular body with a passage therebetween, said tubular body having a forward end connectable to a catheter end and a rear end connectable to tubing, a latch mounted on said adaptor, said latch including a forward portion adapted to cooperate with said catheter end to hold catheter end on said forward end, characterized by:
a base adapted to be mounted on a patient's skin, said base having a pair of upstanding legs spaced apart to receive said adaptor tubular body, said adaptor body and said base legs including engaging structure for releasably holding said adaptor on said base, said structure including a groove and detents, adapted to fit within said groove, to permit said adaptor to rotate a limited amount about the longitudinal axis and said adaptor tubular body while remaining captured on said base.

The base is preferably adapted to be attached to the patient's skin by means of an adhesive pad. The base can be formed integral with the pad or selectively detachable therefrom. The upstanding legs define a space for receiving the adaptor and are constructed to flex outwardly at the upper end to provide a snap fit with the adaptor. Preferably, this snap fit includes an inwardly extending detent on the upper end of each leg. The detents fit within the groove which is annular and formed on the outer surface of the adaptor tubular portion. The detents hold the adaptor to the base and permit the adaptor to rotate from its upright position, when the connection is being made to the connector on the end of a catheter, into a lower profile "stored" position closer to the base and the patient's skin. In this position the components are less likely to interfere with other surrounding actions.

According to a further aspect of the present invention there is provided a method of connecting a catheter to supply tubing comprising:
connecting the forward end of a tubular adaptor to the rear of a catheter hub, with the rear of the tubular adaptor being connected to a supply tubing;
latching the adaptor body to the catheter hub by means of movable latch structure positioned radially outwardly from one side of the tubular adaptor body;
positioning said adaptor body into a base attachable to a patient's skin; and
rotating the adaptor body within said base from a high profile position wherein said latch structure extends upwardly to a low profile position wherein said latch structure extends adjacent to a patient's skin.

Preferably the method includes the steps of engaging said hub with a latch which is slidably mounted on said adaptor;
sliding said latch rearwardly on said adaptor to grip the catheter hub; and
locking said latch in its gripping position by means of an interengaging structure on said latch and adaptor.

Further the forward end of said tubular adaptor body preferably comprises a needle and said catheter hub comprises a septum having a tapering exterior decreasing in diameter in a direction remote from said adaptor body, the method preferably including connecting the rear end of said adaptor body to a supply tubing, and
engaging said septum with the slidable latch mounted on said adaptor body.

The present invention will now be further described, by way of example, with reference to the accompanying drawings, in which:-
Figure 1 is a perspective, schematic view of a typical catheterization installation using the catheter adaptor of the present invention.
Figure 2 is a perspective view of catheterization apparatus of the present invention, mounted on the back of a patient's hand.
Figure 3 is an exploded perspective view of the apparatus of Figure 2.
Figure 4 is a top, partially sectionalized view of the apparatus of Figure 3, illustrating the operation of the resiliently-mounted movable clip and ratchet arrangement, and also illustrating the rotation of the adapter to its low-profile or "stored" position.
Figure 4a is an alternate embodiment of the adapter of Figure 4, illustrating a retention device for securing the adapter in its rotated or stored position.
Figure 5 is a perspective view similar to Figure 3, illustrating the embodiment of the present invention which is compatible with a standard septum-type connector.
Figure 5a illustrates an anchor pad which optionally can be used in combination with the connector of Figure 5 to secure the septum connection to the skin of the patient.
Figures 6 and 6a illustrate another embodiment of the septum connector of Figure 5, illustrating a removable needle cap to prevent accidental self-injection.

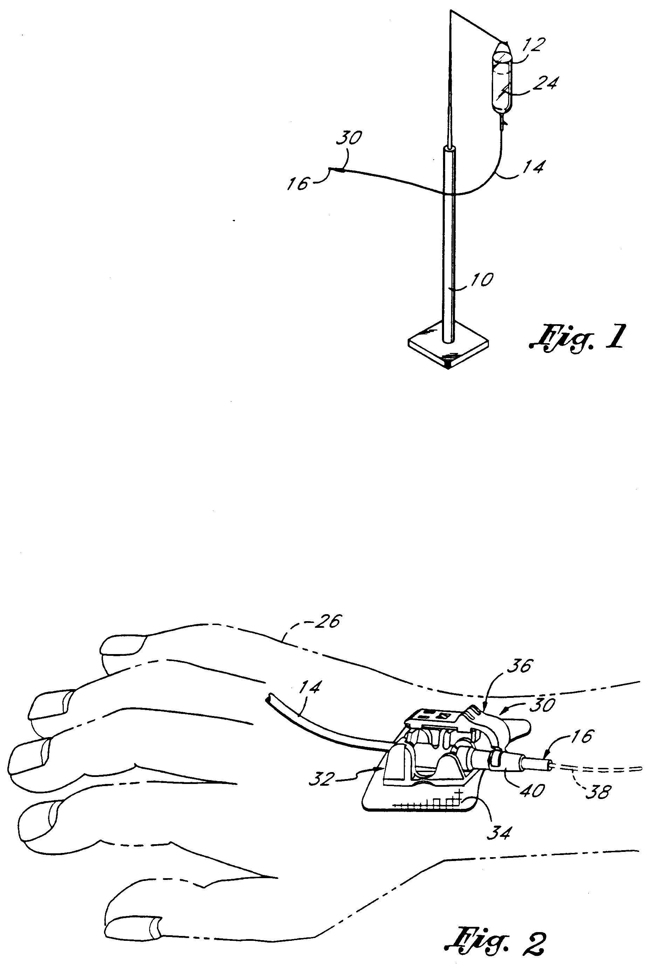

Referring now to Figure 1 there is shown a basic set-up for a catheterization procedure, including a support stand 10, a container 12, a length of IV tubing 14 connected by an adaptor 30 to the actual catheter 16, which is inserted into the patient. Typically, a patient is lying in a bed or is seated adjacent to a stand 10. Fluid 24, to be dispensed into the patient, is maintained in the container 12 and is fed under the pressure of the head of the fluid above the patient, through the IV tubing 14 and catheter 16 into the patient.

### Adapter for Luer-Type Connection

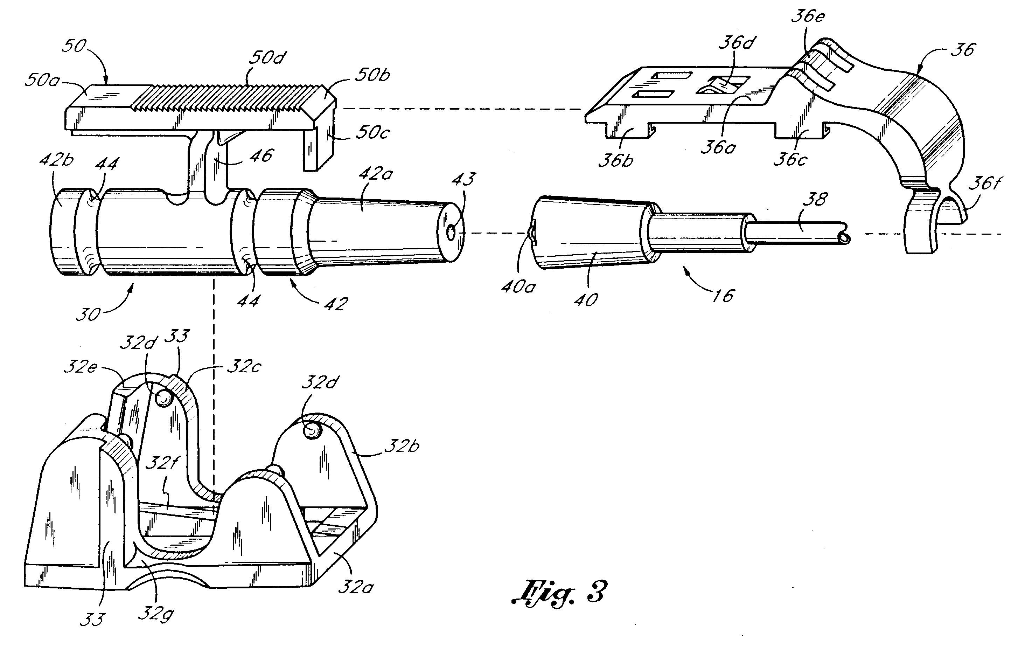

The embodiment of the present invention adapted to be compatible with the standard luer-type connections will first be described in relation to Figures 2-4a. However, it should be pointed out that the principles of the present invention are not limited to either luer- or septum-type connections, but can be utilized with a wide variety of IV connectors, both presently existing and hereinafter arising.

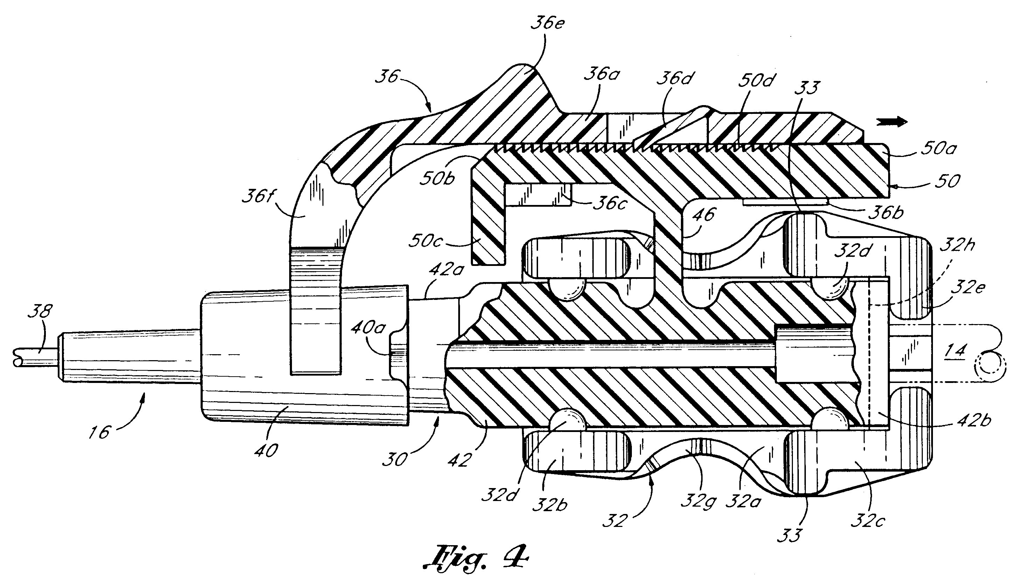

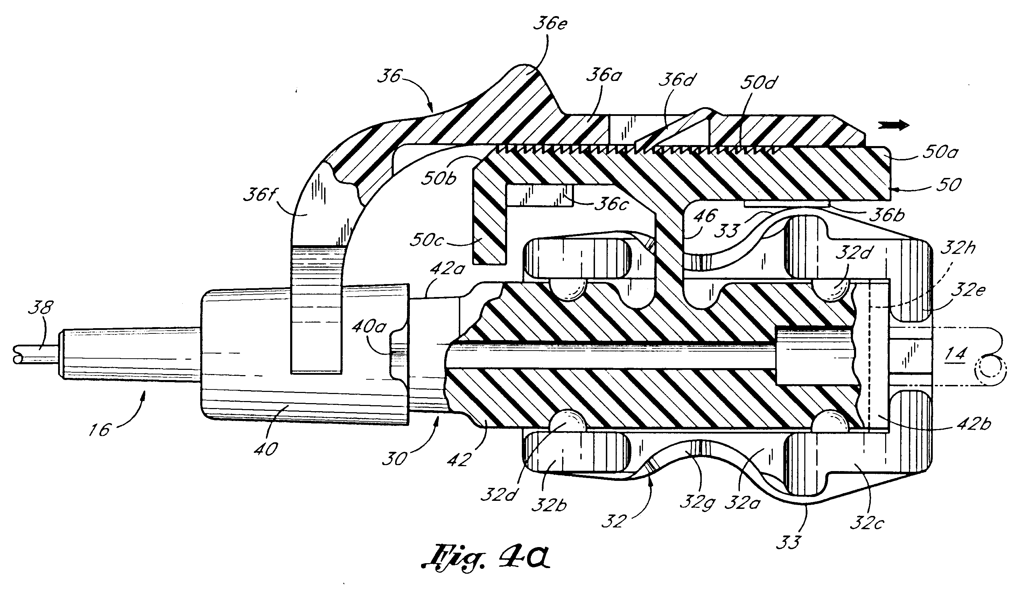

Figure 2 illustrates an enlargement of the catheterization apparatus of the present invention, positioned in the back of a patient's hand 26, shown in phantom lines. As better seen in Figures 3 and 4, the apparatus includes the adaptor 30 interconnecting the catheter 16 and the tubing 14, a base support 32 mounted on a pad 34, and a latching clip 36. For convenience, the pad 34 upon which the base support 32 is integrally mounted is shown only in Figure 2. Such pad 34 could be integral with the base 32 or selectively detachable therefrom. The base can be integrally mounted on the pad 34 in any one of various ways well known in the art, such as adhesive. In the detachable mode, velcro or other well-known means can be used to mount the base on the pad. Furthermore, for clarity of illustration and description, Figure 3 illustrates the latching clip 36 in exploded fashion from the adapter 30; however, it should be understood that, in use, these two elements are initially mated together.

The catheter 16 includes a cannula 38 and a conventional hub 40, having a frusto-conical shape and terminating at its rear end in radially outwardly extending flange portions 40a. Some hubs have a continuous flange.

The adaptor 30 includes a main elongated tubular body 42 having a passage 43 therethrough. The body has a forward end 42a which tapers to fit within an opening in the catheter hub 40. The rear end 42b of the tubular body 42 is adapted to receive the downstream end of the tubing 14. The adaptor further includes a pair of axially spaced annular grooves 44 formed on the exterior of the tubular body. Extending outwardly from the body 42 in cantilever fashion is a support arm 46. Positioned on the radially outer end of the arm 46 is a ratchet element 50 that extends generally parallel to and spaced from the axis of the tubular body 42. The connection to the support arm 46 is between the ends of the ratchet element 50. The adaptor body 42, the arm 46 and the ratchet element 50 are preferably formed as one piece, preferably of a stiff but somewhat flexible plastic. Thus, although the arm 46 and the element 50 are stiff, the element can pivot somewhat about the upper end of the arm, and the arm can flex somewhat about its lower end. Thus depressing the rear end 50a of the ratchet element 50 towards the body 42, will correspondingly move the forward end 50b away from the body 42.

Formed integral with the forward end 50b is a depending stop 50c, which extends toward the body 42 and limits the movement of the forward end 50b towards the tubular body, and prevents fatigue of flexing of the arm 46 and element 50. Formed on the upper or radially outer, flat surface of the ratchet element 50 are a series of ratchet teeth 50d which extend laterally generally parallel to the axis of the tubular body.

The anchor pad 34 is preferably made of a woven cloth material having a self-adhesive backing for attachment to the skin; or alternatively, a foam material with adhesive back. The anchor pad may also be connected to a support for a loop of tubing, and the pad may be specially formed for a left hand or a right hand mounting.

The adaptor base 32 has a generally rectangular lower wall 32a, having its lower surface suitably attached to the top surface of the anchor pad. A pair of spaced upstanding legs 32b are located on the forward end of the base, and a second pair of outwardly extending legs 32c are mounted on the rear portion of the lower wall 32a. These legs define a space sized to receive the tubular body of the adaptor 30. Each leg has on its upper end an inwardly extending detent or nib 32d. The spacing between detents in each pair of legs is slightly less than the smaller diameter of the grooves 44 on the adaptor. Also, the detents 32d on the legs 32b are spaced upwardly from the bottom wall 32a a distance greater than the radius of the adaptor tubular body, and the detents 32d on the legs 32c are spaced above a rib 32f.

The base is made of relatively stiff plastic material but is somewhat flexible, such that the upper ends of the legs, which are supported in cantilever fashion from the base wall 32, can be forced outwardly by the adaptor when it is placed between the legs. When the adaptor is seated, the upper ends of the legs snap inwardly to their original position, such that the adaptor Is securely held within the base. Since the detents 32d are positioned within the annular grooves 44, the adaptor is also securely positioned in its axial direction. The base also includes a rear upstanding wall 32e, which further helps position the adaptor within the base. The base rib 32f is positioned between the legs 32c and extends upwardly to support the adaptor. The upper edge of the rib is angled so that it is higher towards the rear of the base than it is towards the forward portion, with the result that the adaptor is positioned at a desired angle. A horizontal ledge 32h (Figure 4) can be provided on the interior side of the rear wall 42c at the rib height to further support the adaptor in its angled position.

The base 32 includes a waist area 32g on the bottom wall 32a between the front and rear legs. This area extends inwardly from the sides of wall 32a and slightly beneath the adaptor to facilitate gripping the adaptor with a thumb and forefinger.

The clip 36 has a main portion 36a with a generally flat rectangular shape. The clip has positioned on its side edges a forward pair of depending, retainer lugs 36c and a rear pair of depending retainer lugs 36b. These lugs include inwardly extending portions which in cooperation with the lower surface of the main portion 36a define side spaces to receive the ratchet element 50 on the adaptor.

Centrally positioned on the clip is a tongue or pawl 36d supported in cantilever fashion from the main body 36a of the clip. The rear portion of the pawl is formed integral with the body 36a, and the forward end is free and depends downwardly below the lower surface of the body 36a, as best seen in Figure 4. The lower edge of the pawl is a straight line and is adapted to cooperate with the ratchet teeth 50d on the ratchet element 50 of the adaptor.

Positioned slightly forwardly from the tongue is an upwardly extending bump 36e adapted to be engaged by a person's finger or thumb so as to slide the clip 36 on the ratchet element 50. The forward end of the clip curves downwardly and includes a fork-shaped latch 36f adapted to straddle the hub of the catheter. The latch preferably has a curved circular shape that extends more than 180°, such as 190° to 200°. This horseshoe shape enables the latch to slip onto a tapered hub but yet lightly grip it and enables the latch to accommodate a variety of hub shapes and surfaces.

In use, the catheter 16 is installed in conventional fashion. That is, a needle is inserted into the patient's blood vessel; and while holding the needle still, the conventional catheter 16 is slid over the needle and into position within the vessel. The needle is then carefully withdrawn leaving the catheter in position. The forward end 42a of the adaptor 30 is inserted into the catheter hub 40. In accordance with the invention, the clip 36 which has been slidably mounted on the adaptor ratchet element 50 is in a forward position so that it does not interfere with the insertion of the adaptor into the catheter hub. On the other hand, if the clip 36 (and more particularly the forked latch 36f) impedes the interengagement of the hub 40 and the forward end 42a of the adapter 30, the forked latch 36f can be easily removed from the alignment area by depressing the rear end 50a of the ratchet element 50. One of the advantages of the present invention is that this action of operating the latch to facilitate the catheter connection can be easily performed with one hand while the other hand holds the catheter. Whether or not the latch needs to be manipulated, once the hub 40 is engaged on the adapter forward end 42a, the clip 36 is then slid rearwardly causing its forked latch 36f to engage the forward surface of the flange portion 40a on the catheter hub, thereby securely attaching the adaptor to the catheter.

Because conventional catheter hubs have dimensional variations, it is desirable that the clip be adjustable to accommodate the various sizes and yet securely hold the adaptor and the catheter hub together. The clip 36 is easily movable rearwardly on the ratchet element, and the ratchet teeth cooperate with the clip tongue to resist forward movement and hold the clip in the manually selected position.

The adaptor is then snapped into the base 32. In doing this, the adaptor is pressed between the upstanding legs of the base with the detents on the adaptor legs sliding within the annular groove to hold the adaptor in the base. The anchor pad 34 attached to the adaptor base is then positioned in the desired location adjacent to the puncture point in the patient's skin. As noted above, the adaptor and the upper end of the centrally located rib 32f and the ledge 32h in the base causes the forward end of the adaptor to be angled downwardly to be best aligned with the axis of the catheter hub.

To reduce the profile of the clip and the adaptor mounting arm, the adaptor together with the clip can be rotated about 90°, in either direction, to the low profile or "stored" position shown in Figure 4. In other words, Figure 4 is a top plan view similar to Figure 2 except that the latching clip 36 is shown in a rotated position. In this position, the adaptor mounting arm extends between the front base legs 32b and the rear base legs 32c, such that the arm is close to the adaptor base, generally parallel to the patient's skin. The height of the assembly has been reduced to the height of the base 33 and thereby reducing the risk of the assembly interfering with surrounding actions. Once so positioned, the adaptor will normally remain in this position.

In order to ensure that this position is maintained, the retention device illustrated in Figure 4a can be utilized. In this embodiment, the retainer lugs 36b on the clip 36 can be extended so that they engage, in press fit relation, a rib 33 (shown in Figure 3) formed on the legs 32c of the base 32. A similar engagement (not shown) could be utilized by extending retainer lugs 36c so that they engage a similar rib (not shown) formed on the exterior surface of legs 32b. This press fit interconnection causes the adapter body 42, and more particularly the annular grooves 44, to be held more tightly in relation to the detents 32d. Thus, the adapter is securely retained in its rotated or "stored" position until the adapter and its associated tubing is to be removed and replaced. At that time, the press fit or frictional engagement holding the clip 36 in the stored position can be easily overcome by manual manipulation in order to rotate the adapter to its initial position, shown in Figure 2.

In replacing an adaptor and tubing, the procedure is essentially reversed from that set forth above, except that the catheter remains in the patient and the adaptor base remains as positioned. Leaving the catheter in position, of course, minimizes discomfort to the patient, and allowing the adaptor base to remain eliminates irritation to the patient's skin that would be caused by removal and replacement. Also operator time is thus saved during such replacement. In removing the adaptor from the base, the attendant's forefingers and thumb can be placed in the base waist area, also engaging the adaptor. The adaptor is then snapped out of the base with a pinching action. The adaptor is separated from the catheter by depressing the rear of the clip and the clip support element to raise the latch so that the adaptor can be withdrawn rearwardly from the catheter.

### Adapter for Septum-Type Connector

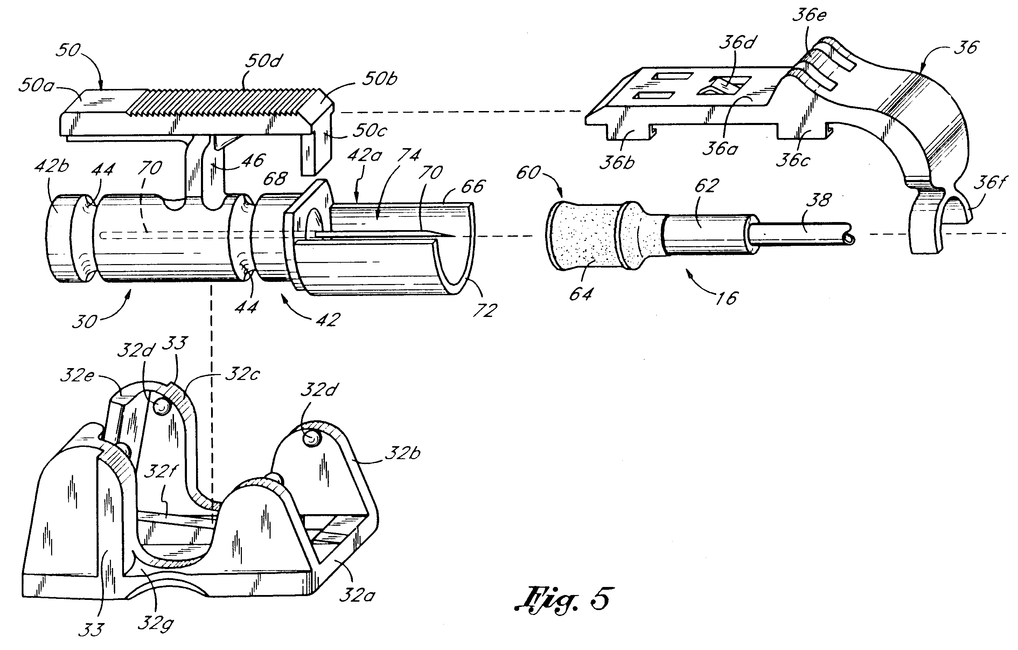

Another embodiment of the present invention comprises an IV adapter which is compatible with standard septum-type connectors. In this context, it is common for a septum-type connection to be utilized in combination with a basic IV catheterization system, as illustrated in Figures 1 and 2, except that the luer-type connector 40 illustrated in Figure 2, mounted on the end of the catheter or cannula 38, is replaced by a septum-type connector 60, as illustrated in Figure 5. This type of connector comprises a cylindrical tube 62, typically constructed from plastic, mounted on the end of the cannula 38, which, in turn, inserts into the vein of the patient. The end of the cylindrical tube 62 is provided by a rubber septum or cap 64 (also referred to as a "buff cop") which is stretched tightly over the end in order to completely close the opening or lumen thereof. Thus, the cap 64 can be perforated by an injection needle in order to administer medication to the patient. When the needle is removed, the rubber material of the cap 64 self-seals in order to prevent leakage.

In use, the septum-type connector is often referred to as a "heparin lock." In order to prevent blood from clotting at the catheter tip, the connector is frequently filled with heparin, which is an anti-coagulant. When the needle is disconnected from the cap, the connector is said to be "locked," i.e., there is no flow.

The adapter 30 of Figure 5 advantageously provides a secure connection with these and similar types of heparin locks or septum-type connectors. For ease of illustration and description, the adapter 30 of Figure 5, like Figure 3, illustrates the clip 36 in exploded relation to the ratchet element 50. However, it will be understood that, in use, prior to connection with the catheter 16, the adapter 30 includes the clip 36 in mated relation to the ratchet element 50 (and typically detached from the base 32).

The adapter 30 of Figure 5 is similar to the adapter of Figure 3, and a description of like elements, indicated by identical reference numerals, will not be repeated; rather, features peculiar to this embodiment shall be described below.

Referring then to Figure 5, the adapter 30 comprises a body 42 having a forward end 42a having a shield 66. The shield mounts on a planar flange 68 which in turn mounts on the end of the tubular body 42 of the adapter 30. It will also be noted that the stop device 50c engages the top of the flange 68 so as to prevent over extension or fatigue of the arm 46. As shown in Figure 5, the shield 66 is partially circular or cylindrical in shape, and extends longitudinally in front of the body 42. Running longitudinally through the body 42 and mounted therein, as shown in dotted lines, is an IV needle 70. The end of an IV tubing (not shown) is then connected to the rear end 42b of the adapter 30 so that fluid is in communication with the IV needle 70. The needle 70 also extends beyond the end of the body 42 and is approximately coaxial with the axis of the shield 66.

Preferably, the distal end 72 of the shield 66 extends slightly beyond the tip of the needle, and the sides of the shield extend above the axis of the needle so as to substantially partially surround the needle. Preferably, the shield 66 circumscribes approximately 180°; however, a circumference of a wide range (such as 160°-220°) would also be within the scope of the invention.

This configuration of the needle 70 and shield 66 shown in Figure 5 provides a high degree of protection from accidental self-injection. The shield provides such protection on substantially three sides of the needle. The only side that is left open is the upper side 74, which advantageously permits improved vision for the user as the adapter 30 is guided into a connection relationship with the cap 64 of the septum. In addition, the shield 66 serves as a guide for those users whose eyesight may not be ideal. Furthermore, the open upper surface 74 of the shield permits the clip 36 to engage the septum, as explained below in more detail. Upon engagement, the shield protects the septum and inhibits contamination. Advantageously, the present invention holds the connection substantially parallel to the skin.

The clip 36 of the adapter 30 serves to securely retain the interconnection between the needle and the septum in a manner similar to that described above in Figures 2-4. Initially, the adapter 30 is typically disengaged from the base 32. The clip 36, although mated with the ratchet element 50, is extended distally therefrom in order to prevent any interference with the septum connection. Because the cap 64 of the septum typically exhibits a larger diameter than standard luer locks or other types of connectors, it may be necessary to elevate the latch 36f in order to avoid interference. This can be done, as described above, by simply depressing the rear end 50a of the ratchet element 50 in order to allow the septum to engage the needle.

It will also be noted in Figure 5 that there is sufficient clearance between the body 42 of the adapter 30 and the rear end 50a of the ratchet element 50 in order to permit the latch 36f to be substantially elevated. Once the septum connection is joined, the clip can be slidably moved toward the rear of the adapter 30 until the latch 36f engages a circumferential portion of the cap, thereby securely retaining the septum connection in its desired position. This secure connection is maintained by the interengagement of the pawl arm 36d and the ratchet members 50d, in a manner described in connection with Figure 4 above. When disconnection is desired, the clip 36 is simply depressed in order to disengage the latch 36f from the septum cap 64. The adapter 30 can then be pulled toward the rear in order to cause the needle 70 to disengage the cap.

It should be noted that, not only is the adapter 30 of the present invention compatible with a variety of IV connectors, it is also compatible with connectors having various diameters and configurations (i.e., unique connectors). Thus, the present invention reduces the cost of typical IV catheterization and facilitates the interchangeability of components.

Figure 5a illustrates an anchor pad 76 which can be optionally utilized with the adapter of Figure 5. Like the anchor pad 34 of Figure 2, the anchor pad 76 illustrated in Figure 5a comprises a base 32 upon which the adapter 30 can be mounted for rotation and retention in a manner similar to that described above. The pad also includes a tubing clip 78 into which a safety loop formed in the IV tubing can be inserted. Forward of the base 32 is a clip 80 in order to retain the cylindrical element 62 of the septum connector 60 (shown in Figure 5). This clip 80 is preferably manufactured from a resilient material, and is thin and flexible in order to retain the cylindrical tube 62 while still permitting slight movement thereof. This combination prevents pain and discomfort to a patient.

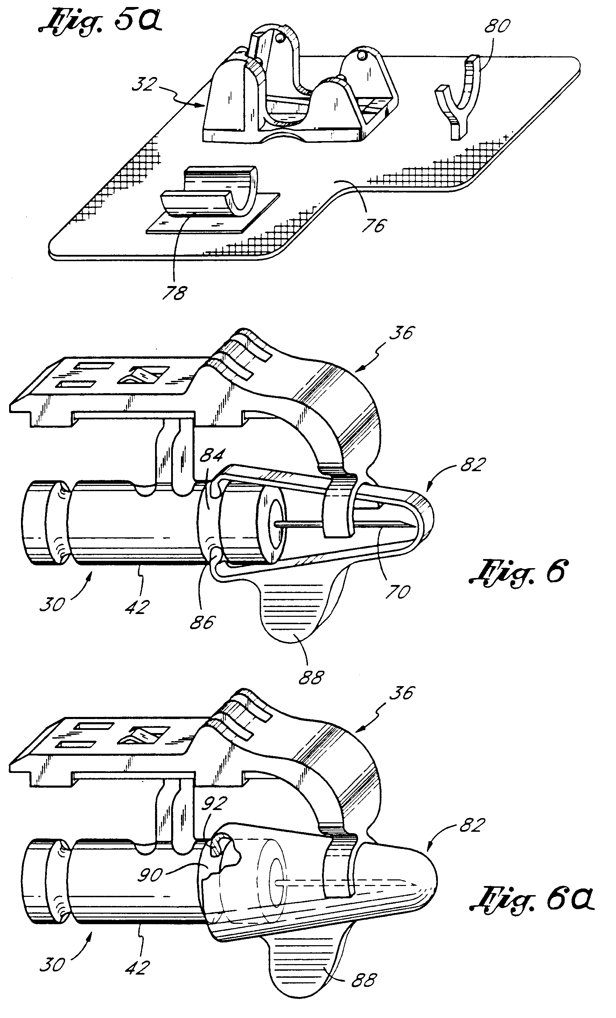

Figures 6 and 6a illustrate an alternate embodiment of an adaptor 30 utilizing a needle cap 82 to prevent accidental self-injection. In this embodiment, the forward end of the body 42 of the adaptor 30 is provided with a pair of substantially parallel grooves 84 formed on the upper and lower sides of the body 42. Mating with the grooves are edges 86 of the needle cap 82. The needle cap is substantially U-shaped in cross-section and rotated approximately 90°, so that when engaged on the end of the adaptor 30, the cap surrounds the needle 70 on two or three sides. Only one side of the cap needs to be left open in order to permit the cap 82 to be slidably engaged in the grooves 84. This cap prevents accidental self-injection when the adaptor is disengaged from the septum-type connector. The cap also includes a lower finger grip 88 to facilitate decapping and recapping, while permitting the fingers to avoid the sharp tip of the needle 70.

In another arrangement, shown in Figure 6a, the needle cap 82 can completely enclose the needle 70 on all sides and be engageable on an annular groove 90 formed on the forward end 42a of the adaptor 30 by means of a press-fit or interference fit with an annular lip 92. Alternatively, the press fit engagement of the cap 82 can be made with the annular groove 44 (Figure 5).

While a preferred arrangement of the catheter mounting apparatus has been illustrated and described, it should be understood that various changes and modifications to the apparatus will readily come to mind and fall within the scope of the invention as set forth in the appended claims.

## Claims

1. An apparatus for connecting a catheter (16) to supply tubing (14), comprising an adaptor (30) having a tubular body (42) with a passage (43) therethrough, the body having a forward end (42a) adapted to connect to a catheter end (40) and a rear end (42b) adapted to be connected to said tubing (14), a latch (36) mounted on said adaptor (30), said latch (36) including a forward portion (36f) adapted to cooperate with said catheter end (40) to hold said catheter end (40) on said forward end (42a), characterized by:
said latch (36) is slidably mounted on said adaptor (30) for movement in a direction generally parallel to an axis through said passage (43), said latch (36) and said adaptor (30) having interengaging structure to hold said latch (36) in a manually selected position on said adaptor (30); and
a base (32) adapted to be mounted on a patient and to support said adaptor (30), said base (32) and said adaptor (30) having structure to releasably secure said adaptor (30) to said base (32).

2. The apparatus of Claim 1, wherein said forward end (42a) is adapted to connect to a hub end of said catheter end (40).

3. The apparatus of Claims 1 or 2, wherein said forward end (42a) comprises a needle (70) to engage a septum connector (60) disposed on said catheter end.

4. The apparatus of Claim 3, additionally comprising an elongated, generally semi-circular shield (72) disposed about said needle (70) proximate to said forward end (42a) to prevent unintentional engagement of said needle (70) with other objects.

5. The apparatus of Claim 3, additionally comprising a needle cap (82) removably covering said needle (70) to prevent accidental self-injection, said needle cap (82) having a U-shaped cross section with respect to a longitudinal axis of said tubular body (42), said needle cap (82) further having and at least two edges (86) at an open end of said needle cap (82), said adaptor body (30) comprising at least two grooves (84) disposed about said forward end (42a) and adapted to engage said needle cap edges (86) to removably secure said needle cap (82) to said adaptor body (30).

6. The apparatus of Claim 5, wherein said needle cap edges form an annular flange (92) circumscribing said open end and said adaptor body grooves form an annular groove (90) positioned to mate with said needle cap annular flange (92).

7. The apparatus of any of Claims 1-6, wherein said interengaging structure includes ratchet teeth (50d) and a pawl (36d) adapted to cooperate with said teeth (50d) to hold said latch (36) in said selected position.

8. The apparatus of any of Claims 1-7, wherein said adaptor (30) includes a support arm (46) extending outwardly from said body (42) and a clip support element (50) mounted on said arm (46), said latch (36) comprising a clip (36) slidably mounted on said element (50), and said forward latch portion (36f) is the forward portion of said clip (36).

9. The apparatus of Claim 8, wherein said interengaging structure includes a plurality of ratchet teeth (50d) on said element, and a pawl (36d) on said clip (36) being flexibly biased against and slidable on said teeth (50d), but cooperating with said teeth (50d) to hold said clip (36) in said selected position.

10. The apparatus of Claims 8 or 9, wherein said interengaging structure includes a generally flat elongated portion (50) and lugs (36b, 36c) which cooperate with the edges of said elongated portion (50) to give the movement of said clip (36) on said element (50).

11. The apparatus of Claim 10, wherein said elongated portion (50) is a part of said element (50) and said lugs (36b, 36c) are mounted on the edges of said clip (36) to slidably fit under the edges of said elongated portion (50).

12. The apparatus of any of Claims 8-11, wherein said structure includes a plurality of ratchet teeth (50d) on the upper surface of said element (50), said teeth (50d) extending generally perpendicular to said tubular portion axis, and said clip (36) having a flexibly mounted pawl (36d) which cooperates with said teeth (50d) to hold the clip (36) in said selected position.

13. The apparatus of any of Claims 8-12, wherein said element (50) and said support arm (46) are constructed in a manner to enable said element (50), together with said clip (36), to be manually pivotable to move said clip forward portion (36a) away from said body forward portion (42a) so as to permit the mounting and demounting of a unique septum connector on said forward end (42a).

14. The apparatus of any of Claims 8-13, wherein said element (50) has a portion with an elongated shape extending generally parallel to said tubular body (42), the central section of said elongated element (50) being mounted in cantilever fashion on the outer end of said arm (46), the connection between said arm (46) and said element (50) permitting pivoting movement of said element (50) on said arm (46) in a manner such that depressing the rear of said element (50) towards said body (42) moves the forward end of said element (50) away from said body (42).

15. The apparatus of any of Claims 8-14, wherein said element (50) includes a depending stop (50c) formed on the forward end of the element (50), the stop (50c) being located to limit the movement of the forward end of said element (50) towards said tubular body (42).

16. The apparatus of any of Claims 8-15, wherein said base (32) and adaptor (30) structure provide a releasable snap-fit engagement that permits said adaptor (30) to be moved about an axis through said body (42) from an operative position wherein said arm (46) extends outwardly on a side remote from said base (32) to a stored position wherein said arm (46) is located close to said base (32).

17. The apparatus of any of Claims 1-16, wherein said base (32) includes a pair of upwardly extending legs (32b) which are positioned to define a space for receiving said adaptor body (42), said legs (32b) each having a detent (32d) on its upper end which extends inwardly into said space, said adaptor body (42) having a groove (44) on its exterior which is adapted to receive said detents (32d), the spacing and location of said detents (32d) in relation to the diameter of said groove (44) being such that the tubular body (42) may be manually pressed into said space and said detents (32d) cooperate with said groove (44) to hold said tubular body (42) in position on said base (32).

18. An apparatus for supporting and facilitating the changing of tubing (14) connected to a catheter, comprising an adaptor (30) having a tubular body (42) with a passage therebetween, said tubular body having a forward end (42a) connectable to a catheter end (40) and a rear end (42b) connectable to tubing (14), a latch (36) mounted on said adaptor (30), said latch (36) including a forward portion (36f) adapted to cooperate with said catheter end (40) to hold catheter end (40) on said forward end (42a), characterized by:
a base (32) adapted to be mounted on a patient's skin, said base having a pair of upstanding legs (32b) spaced apart to receive said adaptor tubular body (42), said adaptor body (42) and said base legs (32b) including engaging structure for releasably holding said adaptor on said base (32), said structure including a groove (44) and detents (32d), adapted to fit within said groove (44), to permit said adaptor (30) to rotate a limited amount about the longitudinal axis and said adaptor tubular body (42) while remaining captured on said base (32).

19. The apparatus of Claim 18, wherein said catheter end comprises a hub of a standard catheter (40).

20. The apparatus of Claim 18, wherein said catheter end (40) comprises a septum connector (60).

21. The apparatus of any of Claims 18-20, wherein said engaging structure includes a detent (32d) formed on the upper end of each of said legs (32b) extending inwardly into said space, and an annular groove (44) formed on the periphery of said adaptor tubular portion sized to receive said detents (32d), the distance between said detents (32d) being slightly less than the diameter of said groove (44), and the detents (32d) being located such that the adaptor (30) can snap into said space with said detents (32d) extending into said groove (44) and holding said adaptor (30) on said base (32) while permitting said adaptor (30) to rotate with respect to said base (32).

22. The apparatus of any of Claims 18-21, wherein said base (32) includes a bottom wall (32a), and said legs (32b) extend upwardly from the bottom wall (32a), said base (32) further includes a second pair of spaced upstanding legs (32c) spaced from the first mentioned pair of legs (32b), and said adaptor (30) includes a support arm (46) attached to and extending generally radially outwardly from said body (42), said adaptor (30) being rotatable from an upright position wherein said arm (46) extends upwardly away from said base bottom wall (32a) and generally parallel to said legs to a low profile position, wherein said arm (46) extends between said pairs of legs adjacent to said bottom wall (32a).

23. The apparatus of any of Claims 18-23, wherein said base (32) includes means between said legs (32b) for supporting said adaptor body (30) so that the rear end of the body (42b) is higher than the forward end (42a) when said body (42) is seated in said base (32).

24. The apparatus of Claim 23, wherein said supporting means comprises an upwardly extending rib (32f) having a sloping upper surface to engage said adaptor body (42).

25. A method of connecting a catheter to supply tubing (14) comprising:
connecting the forward end of a tubular adaptor (42) to the rear of a catheter hub (40), with the rear of the tubular adaptor (42) being connected to a supply tubing (14);
latching the adaptor body (42) to the catheter hub (40) by means of movable latch structure (36) positioned radially outwardly from one side of the tubular adaptor body (42);
positioning said adaptor body (42) into a base (32) attachable to a patient's skin; and
rotating the adaptor body (42) within said base (32) from a high profile position wherein said latch structure (36) extends upwardly to a low profile position wherein said latch structure (36) extends adjacent to a patient's skin.

26. The method of Claim 25, wherein said latching step additionally comprises the steps of:
engaging said catheter hub (40) with said latch (36), wherein said latch (36) is slidably mounted on said adaptor (30);
sliding said latch (36) rearwardly on said adaptor (30) to grip said catheter hub (40); and
locking said latch (36) in its gripping position by means of an interengaging structure on said latch (36) and adaptor (30).

27. The method of Claim 25 or 26, wherein said forward end (42a) of said tubular adaptor body (42) comprises a needle (70) and said catheter hub (40) comprises a septum (60), and said connecting step additionally comprises inserting said needle (70) into said septum (60), and thereafter engaging said septum (60) with said latch (36).

28. The method of Claim 27, additionally comprising the steps of:
providing said latch (36) comprising a clip (36) having a forward latching portion (36f) engaging said catheter septum (60) and a rear portion slidably mounting on said adaptor (30); and
depressing said clip rear portion about a support element (50) on said adaptor (30) to raise said clip forward end (36f) to release said latching portion from said catheter septum (60).

## Patentansprüche

1. Vorrichtung zum Anschließen eines Katheters (16) an den Zuleitungsschlauch (14), die folgendes umfaßt: Einen Adapter (30) mit einem rohrförmigen Körper (42) mit einem Durchgang (43) dort hindurch, wobei der Körper ein vorderes Ende (42a) aufweist, das zum Anschließen an ein Katheterende (40) angepaßt ist, und ein rückwärtiges Ende (42b), das angepaßt ist, damit es an genannten Schlauch (14) angeschlossen werden kann, eine Sperrvorrichtung (36), die an genannten Adapter (30) montiert ist, wobei genannte Sperrvorrichtung (36) einen vorderen Anteil (36f) einschließt, der angepaßt ist, um mit genanntem Katheterende (40) zusammenzuarbeiten, um genanntes Katheterende (40) an genanntem vorderen Ende (42a) festzuhalten, dadurch gekennzeichnet, daß -
genannte Sperrvorrichtung (36) gleitbar an genannten Adapter (30) zur Bewegung in einer Richtung montiert ist, die im allgemeinen parallel zu einer Achse durch genannten Durchgang (43) verläuft, wobei genannte Sperrvorrichtung (36) und genannter Adapter (30) eine ineinandereingreifende Struktur aufweisen, um genannte Sperrvorrichtung (36) in einer manuell gewählten Position an genanntem Adapter (30) zu halten und
ein Unterteil (32), das angepaßt ist, um an einem Patienten befestigt werden zu können und um genannten Adapter (30) zu stützen, wobei genanntes Unterteil (32) und genannter Adapter (30) eine Struktur aufweisen, um genannten Adapter (30) lösbar an genanntes Unterteil (32) sicher zu befestigen.

2. Vorrichtung nach Anspruch 1, worin genanntes vorderes Ende (42a) angepaßt ist, um an ein Ansatzende von genanntem Katheterende (40) angeschlossen werden zu können.

3. Vorrichtung nach Ansprüchen 1 oder 2, worin genanntes vorderes Ende (42a) eine Nadel (70) umfaßt, um in einen Septumkonnektor (60) einzugreifen, der an genanntem Katheterende angeordnet ist.

4. Vorrichtung nach Anspruch 3, die zusätzlich einen länglichen, im allgemeinen halbkreisförmigen Schutzschild (72) umfaßt, der um genannte Nadel (70) herum in Nähe zu genanntem vorderen Ende (42a) angeordnet ist, um das unbeabsichtigte Eingreifen von genannter Nadel (70) in andere Objekte zu verhindern.

5. Vorrichtung nach Anspruch 3, die zusätzlich eine Nadelkappe (82) umfaßt, die genannte Nadel (70) abnehmbar abdeckt, um eine versehentliche Selbstinjektion zu verhindern, wobei genannte Nadelkappe (82) einen U-förmigen Querschnitt in bezug auf eine Längsachse von genanntem rohrförmigen Körper (42) aufweist, wobei genannte Nadelkappe (82) überdies mindestens zwei Kanten (86) an einem offenen Ende von genannter Nadelkappe (82) aufweist, wobei genannter Adapterkörper (30) mindestens zwei Rillen (84) umfaßt, die um genanntes vorderes Ende (42a) herum angeordnet und angepaßt sind, um genannte Nadelkappen-Kanten (86) eingreifen zu lassen, um genannte Nadelkappe (82) an genanntem Adapterkörper (30) abnehmbar sicher zu befestigen.

6. Vorrichtung nach Anspruch 5, worin genannte Nadelkappenkanten einen ringförmigen Flansch (92) bilden, der genanntes offenes Ende umschreibt und genannte Adapterkörperrille eine ringförmige Rille (90) bildet, die so positioniert ist, um mit dem ringförmigen Flansch (92) von genannter Nadelkappe zusammenzupassen.

7. Vorrichtung nach einem der Ansprüche 1 bis 6, worin genannte ineinandereingreifende Struktur Ratschenzähne (50d) und eine Sperrklinke (36d) einschließt, die angepaßt ist, um mit genannten Zähnen (50d) zusammenzuarbeiten, um genannte Sperrvorrichtung (36) in genannter gewählter Position zu halten.

8. Vorrichtung nach einem der Ansprüche 1 bis 7, worin genannter Adapter (30) einen Haltearm (46) einschließt, der von genannten Körper (42) nach außen verläuft und ein Klemmenhalteelement (50), das an genannten Arm (46) montiert ist, wobei genannte Sperrvorrichtung (36) eine Klemme (36) umfaßt, die an genannten Element (50) gleitbar montiert ist und genannter vorderer Sperrvorrichtungsanteil (36f) der vordere Anteil von genannter Klemme (36) ist.

9. Vorrichtung nach Anspruch 8, worin genannte ineinandereingreifende Struktur eine Vielzahl von Ratschenzähnen (50d) an genanntem Element einschließt und wobei eine Sperrklinke (36d) an genannter Klemme (36) flexibel vorgespannt gegen und gleitbar auf genannten Zähnen (50d) ist, aber mit genannten Zähnen (50d) zusammenarbeitet, um genannte Klemme (36) in genannter gewählter Position zu halten.

10. Vorrichtung nach Ansprüchen 8 oder 9, worin genannte ineinandereingreifende Struktur einen im allgemeinen flachen länglichen Anteil (50) und Ansätze (36b, 36c) einschließt, die mit den Kanten von genanntem länglichen Anteil (50) zusammenarbeiten, um die Bewegung von genannter Klemme (36) an genannten Element (50) zu geben.

11. Vorrichtung nach Anspruch 10, worin genannter länglicher Anteil (50) ein Teil von genannten Element (50) ist und genannte Ansätze (36b, 36c) an den Kanten von genannter Klemme (36) montiert sind, um gleitbar unter die Kanten von genanntem länglichen Anteil (50) zu passen.

12. Vorrichtung nach einem der Ansprüche 8 bis 11, worin genannte Struktur eine Vielzahl von Ratschenzähnen (50d) auf der oberen Oberfläche von genanntem Element (50) einschließt, wobei genannte Zähne (50d) im allgemeinen rechtwinklig zu genannter Achse des rohrförmigen Anteils verlaufen und genannte Klemme (36) eine flexibel montierte Sperrklinge (36d) aufweist, die mit genannten Zähnen (50d) zusammenarbeitet, um die Klemme (36) in genannter gewählter Position zu halten.

13. Vorrichtung nach einem der Ansprüche 8 bis 12, worin genanntes Element (50) und genannter Haltearm (46) auf eine Weise konstruiert sind, um genanntes Element (50) zu befähigen, zusammen mit genannter Klemme (36) manuell als Drehpunkt zu dienen, um genannten vorderen Klemmenanteil (36a) von genannten vorderen Körperanteil (42a) wegzubewegen, um das Montieren und Demontieren eines einmaligen Septumkonnektors an genannten vorderen Ende (42a) zu erlauben.

14. Vorrichtung nach einen der Ansprüche 8 bis 13, worin genanntes Element (50) einen Anteil mit einer länglichen Form aufweist, die in allgemeinen parallel zu genannten rohrförmigen Körper (42) verläuft, wobei der zentrale Abschnitt von genannten länglichen Element (50) in Auskragungsart am äußeren Ende von genannten Arm (46) montiert ist, wobei die Verbindung zwischen genannten Arm (46) und genannten Element (50) die Drehbewegung von genannten Element (50) an genannten Arm (46) auf eine Weise erlaubt, daß sich durch Hinunterdrücken des rückwärtigen Anteils des genannten Elementes (50) in Richtung des genannten Körpers (42) das vordere Ende von genanntem Element (50) weg von genannten Körper (42) bewegt.

15. Vorrichtung nach einem der Ansprüche 8 bis 14, worin genanntes Element (50) einen abhängigen Anschlag (50c) einschließt, der an vorderen Ende des Elementes (50) gebildet wird, wobei der Anschlag (50c) so positioniert ist, um die Bewegung des vorderen Endes von genannten Element (50) in Richtung von genannten rohrförmigem Körper (42) zu begrenzen.

16. Vorrichtung nach einen der Ansprüche 8 bis 15, worin genannte Unterteil- (32) und Adapterstruktur (30) einen lösbaren Einschnappeingriff vorsehen, der genannten Adapter (30) erlaubt, sich über eine Achse durch genannten Körper (42) von einer einsatzbereiten Position, worin genannter Arm (46) an einer Seite von genannten Unterteil (32) entfernt nach außen verläuft, an eine nicht einsatzbereite Position zu bewegen, worin genannter Arm (46) dicht an genanntes Unterteil (32) positioniert ist.

17. Vorrichtung nach einem der Ansprüche 1 bis 16, worin genanntes Unterteil (32) ein Paar nach oben verlaufende Beine (32b) einschließt, die so positioniert sind, um einen Raum zur Aufnahme von genannten Adapterkörper (42) zu definieren, wobei genannte Beine (32b) jeweils eine Feststellvorrichtung (32d) an seinem oberen Ende aufweisen, die nach innen in genannten Raum verläuft, wobei genannter Adapterkörper (42) eine Rille (44) auf seiner Außenseite aufweist, die angepaßt ist, um genannte Feststellvorrichtungen (32d) aufzunehmen, wobei der Abstand und die Positionierung von genannten Feststellvorrichtungen (32d) in Relation zum Durchmesser von genannter Rille (44) dergestalt ist, daß der rohrförmige Körper (42) manuell in den genannten Raum gedrückt werden und genannte Feststellvorrichtungen (32d) mit genannter Rille (44) zusammenarbeiten können, um genannten rohrförmigen Körper (42) an genannten Unterteil (32) in Position zu halten.

18. Vorrichtung zur Unterstützung und Erleichterung des Wechselns eines an einen Katheter angeschlossenen Schlauches (14), die folgendes umfaßt: Einen Adapter (30) mit einem rohrförmigen Körper (42) mit einen Durchgang dazwischen, wobei genannter rohrförmiger Körper ein vorderes Ende (42a) aufweist, das an ein Katheterende (40) anschließbar ist und ein rückwärtiges Ende (42b), das an Schlauch (14) anschließbar ist, eine Sperrvorrichtung (36), die an genannten Adapter (30) montiert ist, wobei genannte Sperrvorrichtung (36) einen vorderen Anteil (36f) einschließt, der angepaßt ist, um mit genannten Katheterende (40) zusammenzuarbeiten, um Katheterende (40) an genanntem vorderen Ende (42a) festzuhalten, dadurch gekennzeichnet durch -
ein Unterteil (32), das angepaßt ist, um an der Haut eines Patienten befestigt zu werden, wobei genanntes Unterteil ein Paar hochstehende Beine (32b) aufweist, die mit Zwischenraum voneinander angeordnet sind, um genannten rohrförmigen Adapterkörper (42) aufzunehmen, wobei Adapterkörper (42) und genannte Beine (32b) am Unterteil eine eingreifende Struktur einschließen, um genannten Adapter an genannten Unterteil (32) lösbar festzuhalten, wobei genannte Struktur eine Rille (44) und Feststellvorrichtungen (32d) einschließt, die angepaßt sind, um in genannte Rille (44) zu passen, um genannten Adapter (30) zu gestatten, eine begrenzte Menge um die Längsachse und genannten rohrförmigen Adapterkörper von Adapter (42) herum zu rotieren, während er an genanntem Unterteil (32) befestigt bleibt.

19. Vorrichtung nach Anspruch 18, worin genanntes Katheterende einen Ansatz eines Standardkatheters (40) umfaßt.

20. Vorrichtung nach Anspruch 18, worin genanntes Katheterende (40) einen Septumkonnektor umfaßt (60).

21. Vorrichtung nach einem der Ansprüche 18 bis 20, worin genannte eingreifende Struktur eine Feststellvorrichtung (32d) einschließt, die am oberen Ende von jedem der genannten Beine (32d) gebildet wird, die nach innen in genannten Raum verläuft, und eine ringförmige Rille (44), die an der Peripherie von genannten rohrförmigem Anteil des Adapters gebildet wird, der so zugeschnitten ist, um genannte Feststellvorrichtungen (32d) aufzunehmen, wobei der Abstand zwischen genannten Feststellvorrichtungen (32d) geringgradig kleiner ist als der Durchmesser von genannter Rille (44) und wobei die Feststellvorrichtungen (32d) dergestalt positioniert sind, daß der Adapter (30) in genannten Raum einschnappen kann, wobei die genannten Feststellvorrichtungen (32d) in genannte Rille (44) verlaufen und genannten Adapter (30) auf genanntem Unterteil (32) festhalten, während genannten Adapter (30) ermöglicht wird, in bezug auf genanntes Unterteil zu rotieren (32).

22. Vorrichtung nach einem der Ansprüche 18 bis 21, worin genanntes Unterteil (32) eine Bodenwand (32a) einschließt, und genannte Beine (32b) von der Bodenwand (32a) nach oben verlaufen, wobei genanntes Unterteil (32) überdies ein zweites Paar räumlich voneinander getrennte hochstehende Beine (32c) einschließt, die räumlich von dem ersten erwähnten Paar Beinen (32b) getrennt sind und genannter Adapter (30) einen Haltearm (46) einschließt, der an genannten Körper (42) angebracht ist und im allgemeinen radial nach außen davon verläuft, wobei genannter Adapter (30) von einer aufrechten Position, worin genannter Arm (46) nach oben, weg von genannter Bodenwand des Unterteils (32a) und im allgemeinen parallel zu genannten Beinen verläuft, zu einer flachprofilierten Position rotierbar ist, worin genannter Arm (46) zwischen genannten Beinpaaren, die unmittelbar an genannte Bodenwand (32a) angrenzen, verläuft.

23. Vorrichtung nach einem der Ansprüche 18 bis 23, worin genanntes Unterteil (32) Mittel zwischen genannten Beinen (32b) zum Stützen genannten Adapterkörpers (30) dergestalt einschließt, daß das rückwärtige Ende des Körpers (42b) höher ist als das vordere Ende (42a), wenn genannter Körper (42) in genannten Unterteil (32) sitzt.

24. Vorrichtung nach Anspruch 23, worin genanntes unterstützende Mittel eine nach oben verlaufende Rippe (32f) umfaßt, die eine abfallende obere Oberfläche aufweist, um in genannten Adapterkörper (42) einzugreifen.

25. Verfahren zum Anschließen eines Katheters an Zuleitungsschlauch (14), das folgendes umfaßt:
Anschließen des vorderen Endes eines rohrförmigen Adapters (42) an die Rückseite eines Katheteransatzes (40), wobei die Rückseite des rohrförmigen Adapters (42) an einen Zuleitungsschlauch (14) angeschlossen wird;
Einschnappen des Adapterkörpers (42) in den Katheteransatz (40) mittels einer beweglichen Sperrvorrichtungsstruktur (36), die radial nach außen von einer Seite des rohrförmigen Adapterkörpers (42) positioniert ist;
Positionieren von genannten Adapterkörper (42) in ein Unterteil (32), das an der Haut eines Patienten befestigt werden kann und
Rotieren des Adapterkörpers (42) in genannten Unterteil (32) von einer hochprofilierten Position, worin genannte Sperrvorrichtungsstruktur (36) nach oben verläuft, an eine flachprofilierte Position, worin genannte Sperrvorrichtungsstruktur (36) unmittelbar an die Haut eines Patienten angrenzend verläuft.

26. Verfahren nach Anspruch 25, worin genannter Einschnappschritt zusätzlich die folgenden Schritte umfaßt:
Eingreifen genannten Katheteransatzes (40) in genannte Sperrvorrichtung (36), worin genannte Sperrvorrichtung (36) gleitbar an genannten Adapter (30) montiert ist;
Gleiten genannter Sperrvorrichtung (36) nach hinten auf genannten Adapter (30), um genannten Katheteransatz (40) zu fassen, und
Verriegeln genannter Sperrvorrichtung (36) in ihre Greifposition mittels einer ineinandereingreifenden Struktur an genannter Sperrvorrichtung (36) und Adapter (30).

27. Verfahren nach Anspruch 25 oder 26, worin genanntes vorderes Ende (42a) von genannten rohrförmigem Adapter-Körper (42) eine Nadel (70) umfaßt und genannter Katheteransatz (40) ein Septum (60) umfaßt, und genannter Anschlußschritt zusätzlich das Einsetzen genannter Nadel (70) in genanntes Septum (60) umfaßt und danach Einschnappen von genannten Septum (60) in genannte Sperrvorrichtung (36).

28. Verfahren nach Anspruch 27, das zusätzlich die folgenden Schritte umfaßt:
Vorsehen genannter Sperrvorrichtung (36), die folgendes aufweist: Eine Klemme (36) mit einem vorderen Sperrvorrichtungsanteil (36f), der in genanntes Katheterseptum (60) eingreift und einem rückwärtigen Anteil, der gleitbar auf genannten Adapter (30) montiert ist und
Hinunterdrücken des genannten rückwärtigen Anteils der Klemme über ein Halteelement (50) an genannten Adapter (30), um das vordere Ende von genannter Klemme (36f) anzuheben, um genannten Sperrvorrichtungsanteil von genannten Katheterseptum (60) zu lösen.

## Revendications

1. Dispositif pour connecter un cathéter (16) à un tuyau de distribution (14), comprenant un adaptateur (30) ayant un corps tubulaire (42) avec un passage (43) à l'intérieur, le corps ayant une extrémité avant (42a) adaptée pour être connectée à une extrémité de cathéter (40) et une extrémité arrière (42b) adaptée pour être connectée audit tuyau (14), un dispositif de blocage (36) monté sur ledit adaptateur (30), ledit dispositif de blocage (36) comprenant une partie avant (36f) adaptée pour coopérer avec ladite extrémité de cathéter (40) afin de maintenir ladite extrémité de cathéter (40) sur ladite extrémité avant (42a), caractérisé par :
le fait que ledit dispositif de blocage (36) est monté de manière coulissante sur ledit adaptateur (30) pour permettre un mouvement selon une direction globalement parallèle à un axe à travers ledit passage (43), ledit dispositif de blocage (36) et ledit adaptateur (30) ayant une structure permettant la mise en prise réciproque afin de maintenir ledit dispositif de blocage (36) en une position choisie manuellement sur ledit adaptateur (30) ; et
une base (32) adaptée pour être mise en place sur un patient et pour supporter ledit adaptateur (30), ladite base (32) et ledit adaptateur (30) ayant une structure permettant de fixer de manière amovible ledit adaptateur (30) sur ladite base (32).

2. Dispositif selon la revendication 1, dans lequel ladite extrémité avant (42a) est adaptée pour être connectée à une extrémité femelle de ladite extrémité de cathéter (40).

3. Dispositif selon les revendications 1 ou 2, dans lequel ladite extrémité avant (42a) comprend une aiguille (70) destinée à venir en prise avec un connecteur à membrane (60) disposé sur ladite extrémité de cathéter.

4. Dispositif selon la revendication 3, comprenant en outre une protection allongée, globalement semi-circulaire (72) disposée autour de ladite aiguille (70) à proximité de ladite extrémité avant (42a) afin d'éviter une mise en prise non intentionnelle de ladite aiguille (70) avec d'autres objets.

5. Dispositif selon la revendication 3, comprenant en outre un capuchon d'aiguille (82) recouvrant ladite aiguille (70) de manière amovible, afin d'éviter une auto-injection accidentelle, ledit capuchon d'aiguille (82) ayant une section transversale en U par rapport à un axe longitudinal dudit corps tubulaire (42), ledit capuchon d'aiguille (82) ayant en outre au moins deux bords (86) à une extrémité ouverte dudit capuchon d'aiguille (82), ledit corps d'adaptateur (30) comprenant au moins deux rainures (84) disposées autour de ladite extrémité avant (42a) et adaptées pour mettre en prise lesdits bords du capuchon d'aiguille (86) afin de fixer de manière amovible ledit capuchon d'aiguille (82) audit corps d'adaptateur (30).

6. Dispositif selon la revendication 5, dans lequel lesdits bords du capuchon d'aiguille forment une bride annulaire (92) entourant ladite extrémité ouverte et lesdites rainures du corps d'adaptateur forment une rainure annulaire (90) positionnée pour correspondre à ladite bride annulaire du capuchon d'aiguille (92).

7. Dispositif selon l'une quelconque des revendications 1 à 6, dans lequel ladite structure de mise en prise réciproque comprend des dents d'encliquetage (50d) et un cliquet (36d) adapté pour coopérer avec lesdites dents (50d) afin de maintenir ledit dispositif de blocage (36) à ladite position choisie.

8. Dispositif selon l'une quelconque des revendications 1 à 7, dans lequel ledit adaptateur (30) comprend un bras de support (46) s'étendant vers l'extérieur depuis ledit corps (42) et un élément de support d'attache (50) monté sur ledit bras (46), ledit dispositif de blocage (36) comprenant une attache (36) montée de façon coulissante sur ledit élément (50), et ladite partie avant du dispositif de blocage (36f) est la partie avant de ladite attache (36).

9. Dispositif selon la revendication 8, dans lequel ladite structure de mise en prise réciproque comprend une pluralité de dents d'encliquetage (50d) sur ledit élément, et un cliquet (36d) sur ladite attache (36) qui est sollicité de manière flexible contre lesdites dents (50d) et peut coulisser sur celles-ci, mais qui coopère avec lesdites dents (50d) afin de maintenir ladite attache (36) à ladite position choisie.

10. Dispositif selon les revendications 8 ou 9, dans lequel ladite structure de mise en prise réciproque comprend une partie allongée globalement plate (50) et des pattes (36b, 36c) qui coopèrent avec les bords de ladite partie allongée (50) pour assurer le déplacement de ladite attache (36) sur ledit élément (50).

11. Dispositif selon la revendication 10, dans lequel ladite partie allongée (50) est une partie dudit élément (50) et lesdites pattes (36b, 36c) sont montées sur les bords de ladite attache (36) de manière à pouvoir être reçues de façon coulissante sous les bords de ladite partie allongée (50).

12. Dispositif selon l'une quelconque des revendications 8 à 11, dans lequel ladite structure comprend une pluralité de dents d'encliquetage (50d) sur la surface supérieure dudit élément (50), lesdites dents (50d) s'étendant globalement perpendiculairement audit axe de la partie tubulaire, et ladite attache (36) ayant un cliquet monté de manière flexible (36d) qui coopère avec lesdites dents (50d) afin de maintenir l'attache (36) à ladite position choisie.

13. Dispositif selon l'une quelconque des revendications 8 à 12, dans lequel ledit élément (50) et ledit bras de support (46) sont construits de manière à permettre audit élément (50), ainsi qu'à ladite attache (36), de pouvoir être pivotés manuellement afin d'éloigner ladite partie avant de l'attache (36a) de ladite partie avant du corps (42a) de manière à permettre le montage et le démontage d'un connecteur à membrane unique sur ladite extrémité avant (42a).

14. Dispositif selon l'une quelconque des revendications 8 à 13, dans lequel ledit élément (50) a une partie avec une forme allongée s'étendant globalement parallèlement audit corps tubulaire (42), la section centrale dudit élément allongé (50) étant montée en porte-à-faux sur l'extrémité extérieure dudit bras (46), la connexion entre ledit bras (46) et ledit élément (50) permettant un mouvement de pivot dudit élément (50) sur ledit bras (46) de telle manière qu'en appuyant sur l'arrière dudit élément (50) vers ledit corps (42), on déplace l'extrémité avant dudit élément (50) loin dudit corps (42).

15. Dispositif selon l'une quelconque des revendications 8 à 14, dans lequel ledit élément (50) comprend une butée intégrale (50c) formée à l'extrémité avant de l'élément (50), la butée (50c) étant disposée pour limiter le déplacement de l'extrémité avant dudit élément (50) vers ledit corps tubulaire (42).

16. Dispositif selon l'une quelconque des revendications 8 à 15, dans lequel ladite structure de base (32) et d'adaptateur (30) assure une prise par encliquetage amovible qui permet de déplacer ledit adaptateur (30) autour d'un axe traversant ledit corps (42) entre une position de fonctionnement dans laquelle ledit bras (46) s'étend vers l'extérieur à l'opposé du côté de ladite base (32) et une position de stockage dans laquelle ledit bras (46) est situé proche de ladite base (32).

17. Dispositif selon l'une quelconque des revendications 1 à 16, dans lequel ladite base (32) comprend une paire de montants s'étendant vers le haut (32b) qui sont positionnés de manière à définir un espace pour recevoir ledit corps d'adaptateur (42), lesdits montants (32d) ayant chacun une butée (32d) sur son extrémité supérieure qui s'étend vers l'intérieur dans ledit espace, ledit corps d'adaptateur (42) ayant une rainure (44) sur sa surface extérieure qui est adaptée pour recevoir lesdites butées (32d), l'espacement et l'emplacement desdites butées (32d) par rapport au diamètre de ladite rainure (44) étant tels que le corps tubulaire (42) peut être manuellement forcé dans ledit espace et lesdites butées (32d) coopèrent avec ladite rainure (44) pour maintenir ledit corps tubulaire (42) en position sur ladite base (32).

18. Dispositif pour supporter et faciliter le remplacement du tuyau (14) connecté à un cathéter, comprenant un adaptateur (30) ayant un corps tubulaire (42) avec un passage à l'intérieur, ledit corps tubulaire ayant une extrémité avant (42a) pouvant être connectée à une extrémité de cathéter (40) et une extrémité arrière (42b) pouvant être connectée à un tuyau (14), un dispositif de blocage (36) monté sur ledit adaptateur (30), ledit dispositif de blocage (36) comprenant une partie avant (36f) adaptée pour coopérer avec ladite extrémité de cathéter (40) afin de maintenir l'extrémité de cathéter (40) sur ladite extrémité avant (42a), caractérisé par :
une base (32) adaptée pour être mise en place sur la peau d'un patient, ladite base ayant une paire de montants s'étendant vers le haut (32b) espacés de manière à recevoir ledit corps tubulaire de l'adaptateur (42), ledit corps d'adaptateur (42) et lesdits montants de la base (32b) comprenant une structure de mise en prise destinée à maintenir ledit adaptateur sur ladite base (32) de manière amovible, ladite structure comprenant une rainure (44) et des butées (32d), adaptées pour être reçues dans ladite rainure (44), afin de permettre la rotation dudit adaptateur (30) dans une certaine mesure autour de l'axe longitudinal dudit corps tubulaire de l'adaptateur (42) tout en restant maintenu sur ladite base (32).

19. Dispositif selon la revendication 18, dans lequel ladite extrémité de cathéter comprend une partie femelle d'un cathéter classique (40).

20. Dispositif selon la revendication 18, dans lequel ladite extrémité de cathéter (40) comprend un connecteur à membrane (60).

21. Dispositif selon l'une quelconque des revendications 18 à 20, dans lequel ladite structure de mise en prise comprend une butée (32d) formée sur l'extrémité supérieure de chacun desdits montants (32b) qui s'étend vers l'intérieur dans ledit espace, et une rainure annulaire (44) formée sur la périphérie de ladite partie tubulaire de l'adaptateur dimensionnée de manière à recevoir lesdites butées (32d), la distance entre lesdites butées (32d) étant légèrement inférieure au diamètre de ladite rainure (44), et les butées (32d) étant situées de manière à ce que l'adaptateur (30) puisse s'encliqueter dans ledit espace avec lesdites butées (32d) s'étendant dans ladite rainure (44), et maintenant ledit adaptateur (30) sur ladite base (32) tout en permettant la rotation dudit adaptateur (30) par rapport à ladite base (32).

22. Dispositif selon l'une quelconque des revendications 18 à 21, dans lequel ladite base (32) comprend une paroi inférieure (32a), et lesdits montants (32b) s'étendent vers le haut depuis la paroi inférieure (32a), ladite base (32) comprend en outre une deuxième paire de montants espacés s'étendant vers le haut (32c) espacés de ladite paire de montants (32b) mentionnée en premier, et ledit adaptateur (30) comprend un bras de support (46) fixé audit corps (42) et s'étendant globalement radialement vers l'extérieur depuis celui-ci, ledit adaptateur (30) pouvant tourner d'une position verticale, dans laquelle ledit bras (46) s'étend vers le haut depuis ladite paroi inférieure de la base (32a) et globalement parallèlement auxdits montants, à une position de profil aplati, dans laquelle ledit bras (46) s'étend entre lesdites paires de montants à proximité de ladite paroi inférieure (32a).

23. Dispositif selon l'une quelconque des revendications 18 à 23, dans lequel ladite base (32) comprend des moyens entre lesdits montants (32b) destinés à supporter ledit corps d'adaptateur (30) de manière à ce que l'extrémité arrière du corps (42b) soit plus haute que l'extrémité avant (42a) lorsque ledit corps (42) est logé dans ladite base (32).

24. Dispositif selon la revendication 23, dans lequel lesdits moyens de support comprennent une nervure s'étendant vers le haut (32f) ayant une surface supérieure en pente destinée à venir en prise avec ledit corps d'adaptateur (42).

25. Procédé pour connecter un cathéter à un tuyau de distribution (14) comprenant le fait de :
connecter l'extrémité avant d'un adaptateur tubulaire (42) à l'arrière d'une partie femelle de cathéter (40), l'arrière de l'adaptateur tubulaire (42) étant connecté à un tuyau de distribution (14) ;
bloquer le corps d'adaptateur (42) sur la partie femelle du cathéter (40) au moyen d'une structure de blocage mobile (36) positionnée radialement vers l'extérieur depuis un côté du corps d'adaptateur tubulaire (42) ;
positionner ledit corps d'adaptateur (42) dans une base (32) qui peut être fixée sur la peau d'un patient ; et
faire tourner le corps d'adaptateur (42) dans ladite base (32) depuis une position à profit haut dans laquelle ladite structure de blocage (36) s'étend vers le haut vers une position à profit bas dans laquelle ladite structure de blocage (36) s'étend à proximité de la peau du patient.

26. Procédé selon la revendication 25, dans lequel ladite étage de blocage comprend en outre les étapes consistant à :
mettre en prise ladite partie femelle de cathéter (40) avec ledit dispositif de blocage (36), dans lequel ledit dispositif de blocage (36) est monté de manière coulissante sur ledit adaptateur (30) ;
faire coulisser ledit dispositif de blocage (36) vers l'arrière sur ledit adaptateur (30) pour serrer ladite partie femelle de cathéter (40) ; et
bloquer ledit dispositif de blocage (36) à sa position de serrage au moyen d'une structure de mise en prise réciproque sur ledit dispositif de blocage (36) et ledit adaptateur (30).

27. Procédé selon la revendication 25 ou 26, dans lequel ladite extrémité avant (42a) dudit corps d'adaptateur tubulaire (42) comprend une aiguille (70) et ladite partie femelle de cathéter (40) comprend une membrane (60), et ladite étape de connexion comprend en outre le fait d'introduire ladite aiguille (70) dans ladite membrane (60), et de mettre ensuite ladite membrane (60) en prise avec ledit dispositif de blocage (36).

28. Procédé selon la revendication 27, comprenant en outre les étapes consistant à :
fournir ledit dispositif de blocage (36) comprenant une attache (36) ayant une partie de blocage avant (36f) venant en prise avec ladite membrane de cathéter (60) et une partie arrière montée de manière coulissante sur ledit adaptateur (30) ; et
abaisser ladite partie arrière de l'attache autour d'un élément de support (50) sur ledit adaptateur (30) pour relever ladite extrémité avant de l'attache (36f) afin de dégager ladite partie de blocage de ladite membrane de cathéter (60).
